(19) Europäisches Patentamt
European Patent Office
Office européen des brevets

(11) Numéro de publication: **0 251 858**
**A1**

(12) # DEMANDE DE BREVET EUROPEEN

(21) Numéro de dépôt: **87401352.7**

(22) Date de dépôt: **17.06.87**

(51) Int. Cl.[4]: **C 07 D 233/61**
A 61 K 31/415

(30) Priorité: **27.06.86 FR 8609331**

(43) Date de publication de la demande:
**07.01.88 Bulletin 88/01**

(84) Etats contractants désignés:
**AT BE CH DE ES FR GB GR IT LI LU NL SE**

(71) Demandeur: **SYNTHELABO**
**58 rue de la Glacière**
**F-75013 Paris (FR)**

(72) Inventeur: **Comarmond, Jacques**
**10, Villa d'Este**
**F-75013 Paris (FR)**

**Purcell, Thomas**
**47bis, rue de la Millière Les Mesnuls**
**F-78490 Montfort L'Amaury (FR)**

**Zard, Lydia**
**6, Impasse des Quatre Vents La Croix Audierne**
**F-91190 Gif S/Yvette (FR)**

(74) Mandataire: **Ludwig, Jacques et al**
**SYNTHELABO Service Brevets 58, rue de la Glacière**
**F-75013 Paris (FR)**

(54) **N-imidazolylméthyl-diphénylazo-méthines, leur préparation et leur application en thérapeutique.**

(57) Composé répondant à la formule générale I

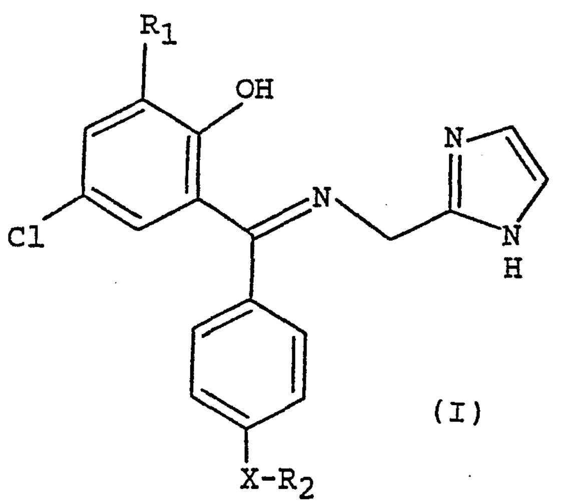

(I)

dans laquelle
$R_1$ représente un atome de chlore ou un groupe méthyle,
X représente un groupe thio ou sulfonyle, et
$R_2$ représente un groupe alkyle en $C_1$-$C_4$, allyle, cyclohexyle, pyridinyle-2, phényle éventuellement substitué par un groupe méthyle ou par un ou deux atomes d'halogène, ou encore benzyle éventuellement substitué par un atome d'halogène, par un groupe méthyle ou par un groupe méthoxy,
ainsi que ses sels d'addition à des acides acceptables en pharmacologie.
Application en thérapeutique.

EP 0 251 858 A1

## Description

N-IMIDAZOLYLMETHYL-DIPHENYLAZOMETHINES, LEUR PREPARATION ET LEUR APPLICATION EN THERAPEUTIQUE

La présente invention a pour objet des N-imidazolylméthyl-diphénylazométhines, leur préparation et leur application en thérapeutique.

Les composés de l'invention répondent à la formule générale I

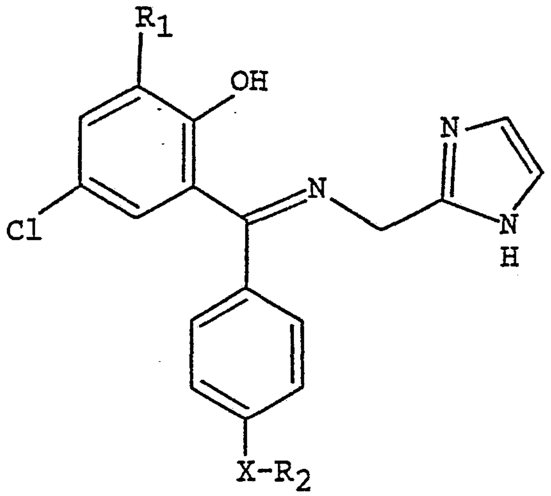

(I)

dans laquelle

$R_1$ représente un atome de chlore ou un groupe méthyle,

X représente un groupe thio ou sulfonyle, et

$R_2$ représente un groupe alkyle en $C_1$-$C_4$, allyle, cyclohexyle, pyridinyle-2, phényle éventuellement substitué par un groupe méthyle ou par un ou deux atomes d'halogène, ou encore benzyle éventuellement substitué par un atome d'halogène, par un groupe méthyle ou par un groupe méthoxy,

ainsi que leurs sels d'addition à des acides acceptables en pharmacologie.

Parmi les composés préférés de l'invention on peut citer ceux dans la formule desquels X représente un groupe sulfonyle et $R_2$ représente un groupe benzyle, en particulier lorsque $R_1$ représente un atome de chlore, ainsi que ceux dans la formule desquels X représente un groupe thio et $R_2$ représente un groupe pyridinyle-2.

La préparation des composés de formule I peut s'effectuer par exemple selon le schéma de la page suivante.

On fait réagir d'abord une benzophénone de formule II avec le sel de sodium d'un mercaptan de formule $R_2$-SH, $R_2$ étant tel

SCHEMA

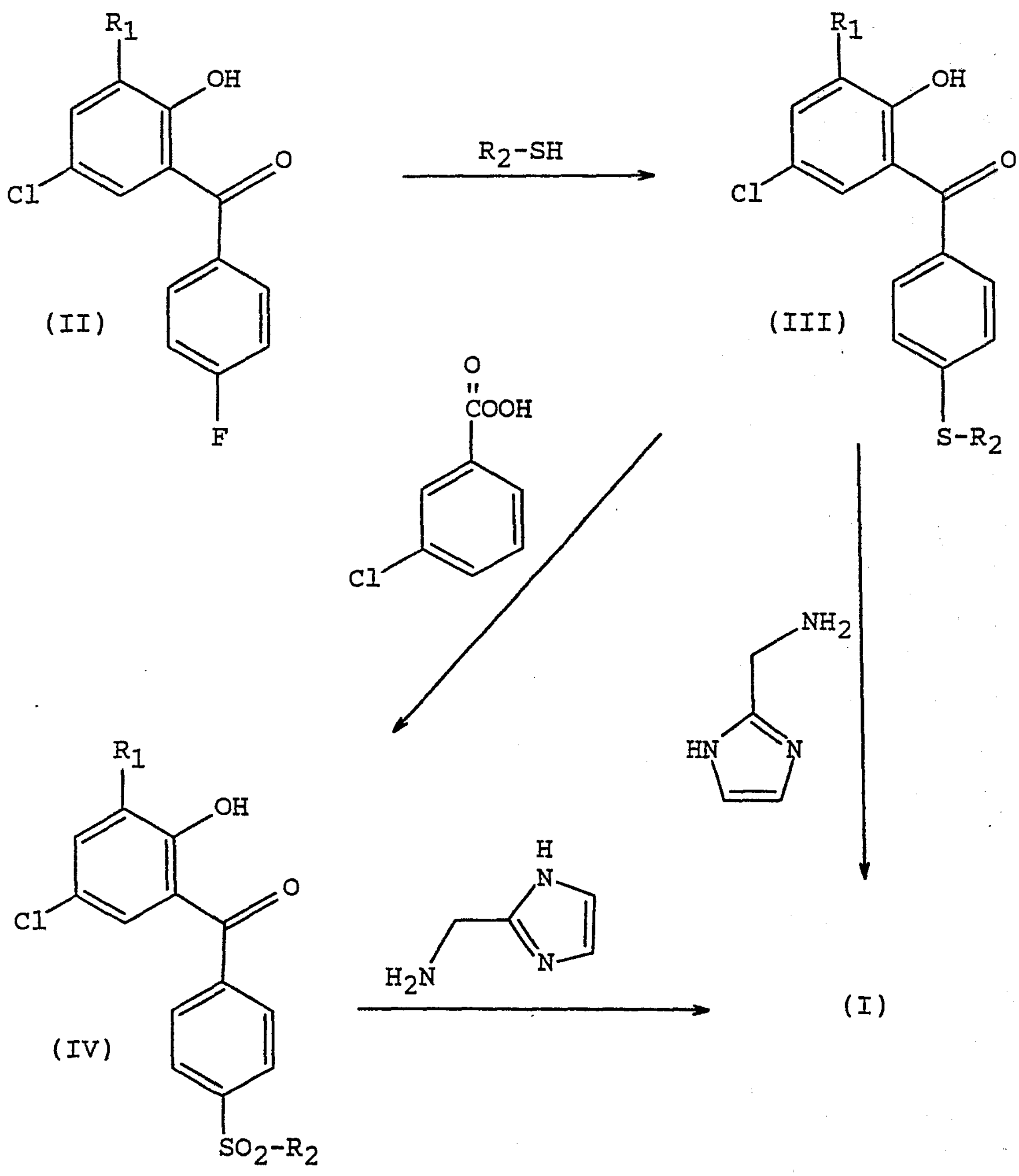


(II) (III) (IV) (I)

que défini ci-dessus, à chaud et dans un solvant tel que le diméthylsulfoxyde, puis, si on désire un composé (I) où X représente un groupe sulfonyle, on oxyde l'intermédiaire de formule III, par exemple au moyen d'acide

chloro-3 perbenzoïque, et finalement on condense le composé de formule III ou le composé oxydé de formule IV avec la (1H-imidazolyl-2-)-méthanamine, à chaud et dans un solvent tel que l'éthanol. Les benzophénones (II) de départ sont connues et peuvent être préparées par exemple selon un procédé décrit dans le brevet britannique nº 2 111 051.

Les exemples qui suivent illustrent en détail le préparation des composés de l'invention. Les microanalyses et les spectres IR et RMN ont confirmé les structures des produits obtenus.

Exemple 1. [(Benzylsulfonyl-4 phényl) [[(1H-imidazolyl-2)méthyl]imino]méthyl]-2 dichloro-4,6 phénol.

1.1. (Benzylthio-4 phényl) (dichloro-3,5 hydroxy-2 phényl méthanone.

On place 5,2 g de sodium dans 250 ml d'éthanol, on ajoute 13,2 ml d'acool thiobenzylique, on agite le mélange 30 minutes et on chasse le solvant.

On dissout le résidu avec 200ml de diméthylsulfoxyde, on ajoute 32 g de (dichloro-3,5 hydroxy-2 phényl) (fluoro-4 phényl) méthanone et on chauffe le mélange à 100°C pendant 8 heures sous argon.

On évapore le diméthylsulfoxyde, on reprend le résidu dans du dichlorométhane et on lave la solution avec de l'acide chlorhydrique à 10 %. On sépare la phase organique, on la sèche sur sulfate de magnésium et on l'évapore.

On purifie le résidu par chromatographie sur silice en éluant avec du dichlorométhane, et on recristallise la fraction purifiée dans le cyclohexane.

Point de fusion : 102-103°C.

1.2. (Benzylsulfonyl-4 phényl)(dichloro-3,5 hydroxy-2 phényl méthanone.

On dissout 22 g de la cétone préparée selon 1.1. dans 600 ml de dichlorométhane, on ajoute 24,4 g d'acide chloro-3 perbenzoïque, et on agite le mélange pendant 1 heure.

Ensuite on filtre le mélange, en lavant le précipité avec du dichlorométhane, et on évapore le filtrat presque à sec.

On purifie le résidu par chromatographie sur silice en éluant avec du chloroforme, on recristallise la fraction purifiée dans l'éthanol.

Point de fusion : 153-154°C.

1.3. [(Benzylsulfonyl-4 phényl) [[(1H-imidazolyl-2)méthyl]-imino]méthyl]-2 dichloro-4,6 phénol.

Dans 350 ml d'éthanol on introduit 3,5 g de dichlorhydrate de (1H-imidazolyl-2)méthanamine et 3,5 g de bicarbonate de sodium, et on chauffe le mélange au reflux pendant 30 minutes. On ajoute ensuite 6 g de la cétone préparée selon 1.2. et on laisse chauffer encore 3 heures au reflux.

On sépare les sels par filtration, on évapore l'éthanol, on purifie le résidu par chromatographie sur silice en éluant avec un mélange 95/5 de dichlorométhane/méthanol, et on recristallise la fraction purifiée dans l'éthanol.

Point de fusion : 197-198°C.

Exemple 2. [(Ethylsulfonyl-4 phényl) [[(1H-imidazolyl-2)méthyl]imino]méthyl]-2 dichloro-4,6 phénol.

2.1. (Ethylthio-4 phényl) (dichloro-3,5 hydroxy-2 phényl méthanone.

On place 1,30 g de sodium dans 200 ml d'éthanol, on ajoute 2 ml d'éthanethiol, on agite le mélange 15 minutes et on chasse le solvant.

On dissout le résidu avec 200 ml de diméthylsulfoxyde, on ajoute 8 g de (dichloro-3,5 hydroxy-2 phényl)(fluoro-4 phényl) méthanone et on chauffe le mélange à 100°C pendant 8 heures sous argon.

On évapore le diméthylsulfoxyde, on reprend le résidu dans du dichlorométhane et on lave la solution avec de l'acide chlorhydrique à 10 %. On sépare la phase organique, on la sèche sur sulfate de magnésium et on l'évapore.

On purifie le résidu par chromatographie sur silice en éluant avec un mélange 40/60 de dichlorométhane/cyclohexane, et on recristallise la fraction purifiée dans le cyclohexane.

Point de fusion : 87-89°C.

2.2. (Ethylsulfonyl-4 phényl)(dichloro-3,5 hydroxy-2 phenyl méthanone.

On dissout 10 g de la cétone préparée selon 2.1. dans 250 ml de dichlorométhane, on ajoute 13 g d'acide chloro-3 perbenzoïque, et on agite le mélange pendant 1 heure.

Ensuite on filtre le mélange, en lavant le précipité avec du dichlorométhane, et on évapore le filtrat presque à sec.

On purifie le résidu par chromatographie sur silice en éluant avec du dichlorométhane, on recristallise la fraction purifiée dans l'éthanol.

Point de fusion : 120-121°C.

2.3. [(Ethylsulfonyl-4 phényl) [[(1H-imidazolyl-2)méthyl]-imino]méthyl]-2 dichloro-4,6 phénol.

Dans 300 ml d'éthanol on introduit 3 g de dichlorhydrate de (1H-imidazolyl-2)méthanamine et 3 g de bicarbonate de sodium, et on chauffe le mélange au reflux pendant 30 minutes. On ajoute ensuite 5 g de la

cétone préparée selon 2.2. et on laisse chauffer encore 3 heures au reflux.
On sépare les sels par filtration, on évapore l'éthanol, on purifie le résidu par chromatographie sur silice en éluant avec un mélange 98/2 de dichlorométhane/méthanol, et on recristallise la fraction purifiée dans l'éthanol.
Point de fusion : 194-195°C.

Le tableau suivant illustre les structures et propriétés physiques de quelques composés selon l'invention.

Tableau

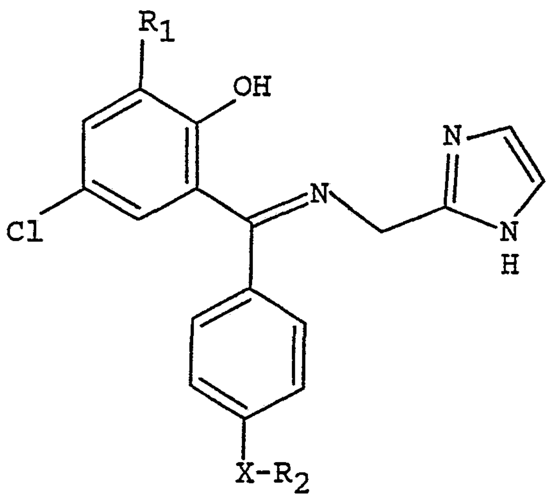

(I)

| Composé | $R_1$ | X | $R_2$ | F(°C) |
| --- | --- | --- | --- | --- |
| 1 | Cl | S | éthyle | 190-191 |
| 2 | Cl | S | n-propyle | 146-148 |
| 3 | Cl | S | i-propyle | 191-193 |
| 4 | Cl | S | allyle | 155-157 |
| 5 | Cl | S | cyclohexyle | 177-178 |
| 6 | Cl | S | pyridinyle-2 | 209-210 |
| 7 | Cl | S | phényle | 199-201 |
| 8 | Cl | S | méthyl-4 phényle | 194-196 |
| 9 | Cl | S | chloro-4 phényle | 184-186 |
| 10 | Cl | S | dichloro-3,4 phényle | 187-188 |
| 11 | Cl | S | benzyle | 175-176 |
| 12 | $CH_3$ | S | benzyle | 178-180 |
| 13 | Cl | S | chloro-4 benzyle | 172-174 |
| 14 | Cl | S | méthoxy-4 benzyle | 186-187 |

Tableau (suite)

| Composé | $R_1$ | X | $R_2$ | F(°C) |
|---|---|---|---|---|
| 15 | Cl | $SO_2$ | éthyle | 194-195 |
| 16 | Cl | $SO_2$ | n-propyle | 195-196 |
| 17 | Cl | $SO_2$ | i-propyle | 192-193 |
| 18 | Cl | $SO_2$ | cyclohexyle | 222-224 |
| 19 | Cl | $SO_2$ | phényle | 217-218 |
| 20 | Cl | $SO_2$ | méthyl-4 phényle | 235-237 |
| 21 | Cl | $SO_2$ | benzyle | 197-198 |
| 22 | $CH_3$ | $SO_2$ | benzyle | 212-214 |
| 23 | Cl | $SO_2$ | chloro-4 benzyle | 227-228 |
| 24 | Cl | $SO_2$ | méthoxy-4 benzyle | 208-210 |

Les composés de l'invention ont été testés en pharmacologie. Leur activité antiulcéreuse a été montrée dans les tests de l'ulcère de stress et de l'ulcère induit par la phénylbutazone.

Ulcère de stress

La technique utilisée est celle de Senay et Levine, Proc. Soc. Exp. Biol. 1967, 124, 1221-1223, Peptic Ulcers. Edited by C.J. PFEIFER. p. 92-97, sur des rats Wistar femelles pesant 180-210 g, à jeun depuis 20 heures, répartis en blocs randomisés.
Les animaux sont mis en contention dans des boîtes cylindriques de 20 cm x 5 cm et placés dans une chambre froide dont la température est maintenue à 2-4°C.
Les composés à étudier sont administrés p.o. à raison de 10, 30 et 100 mg/kg immédiatement avant la mise en contention, les rats témoins recevant seulement le placébo.
2 heures plus tard, les animaux sont sacrifiés par inhalation de chloroforme.
Les estomacs sont prélevés et le degré d'ulcération est noté. Les composés de l'invention diminuent significativement (jusqu'à 83 % à la dose de 30 mg/kg) les ulcères de stress.

Ulcère induit par la phénylbutazone

Le test est effectué sur des rats Wistar femelles pesant 180-210 g à jeun depuis 20 heures, répartis en blocs randomisés. Les ulcères sont provoqués par l'administration orale de phénylbutazone en solution mole à mole avec de la soude, à la dose de 200 mg/kg.
Les composés à étudier sont administrés p.o. à raison de 10, 30 et 100 mg/kg, 30 minutes avant l'ingestion de phénylbutazone, les animaux témoins ne recevant que le placébo.
Deux heures après l'administration de l'agent ulcérigène, les animaux sont sacrifiés par inhalation de chloroforme. Les estomacs sont prélevés et le degré d'ulcération est noté. Les composés de l'invention diminuent significativement (jusqu'à 98 % à la dose de 30 mg/kg) les ulcères induits par la phénylbutazone.
Les composés de l'invention peuvent donc être utilisés pour le traitement des ulcères gastriques, duodénaux ou gastroduodénaux.
Ils peuvent être administrés par voie orale ou parentérale, sous la forme de compositions pharmaceutiques contenant la substance active en association avec tout excipient approprié, par exemple sous la forme de comprimés, dragées, gélules, capsules ou de solutions ou de suspensions buvables ou injectables.
La dose journalière peut être de 10 à 2000 mg de substance active chez l'homme.

**Revendications**

1. Composé répondant à la formule générale I

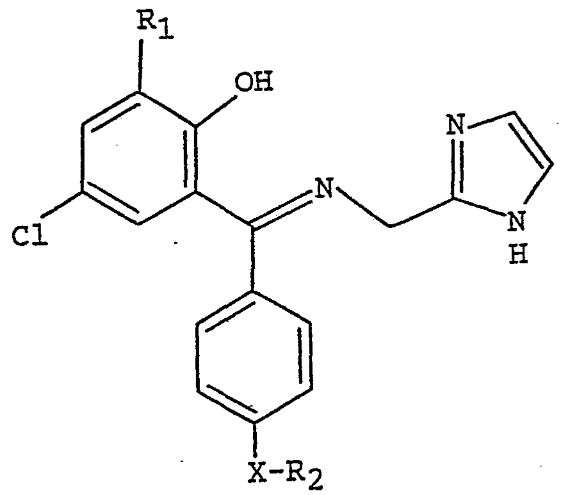

(I)

dans laquelle
$R_1$ représente un atome de chlore ou un groupe méthyle,
X représente un groupe thio ou sulfonyle, et
$R_2$ représente un groupe alkyle en $C_1$-$C_4$, allyle, cyclohexyle, pyridinyle-2, phényle éventuellement substitué par un groupe méthyle ou par un ou deux atomes d'halogène, ou encore benzyle éventuellement substitué par un atome d'halogène, par un groupe méthyle ou par un groupe méthoxy,
ainsi que ses sels d'addition à des acides acceptables en pharmacologie.

2. Composé selon la revendication 1, caractérisé en ce que X représente un groupe sulfonyle et $R_2$ représente un groupe benzyle.

3. Composé selon la revendication 2, caractérisé en ce que $R_1$ représente un atome de chlore.

4. Composé selon la revendication 2, caractérisé en ce que $R_1$ représente un groupe méthyle.

5. Composé selon la revendication 1, caractérisé en ce que X représente un groupe thio et $R_2$ représente un groupe pyridinyle-2.

6. Procédé de préparation d'un composé selon la revendication 1, caractérisé en ce qu'on fait réagir une benzophénone de formule II

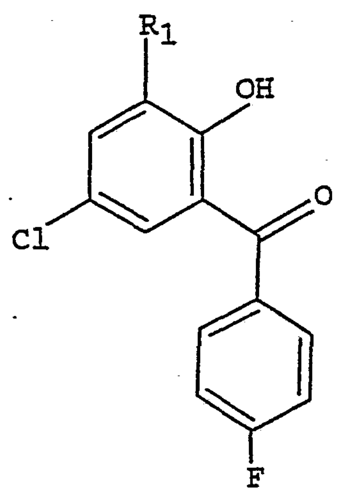

(II)

dans laquelle $R_1$ est tel que défini dans la revendication 1, avec le sel de sodium d'un mercaptan de formule $R_2$-SH, dans laquelle $R_2$ est tel que défini dans la revendication 1, à chaud et dans un solvant tel que le diméthylsulfoxyde, puis, si on désire un composé (I) où X représente un groupe sulfonyle, on oxyde l'intermédiaire de formule III

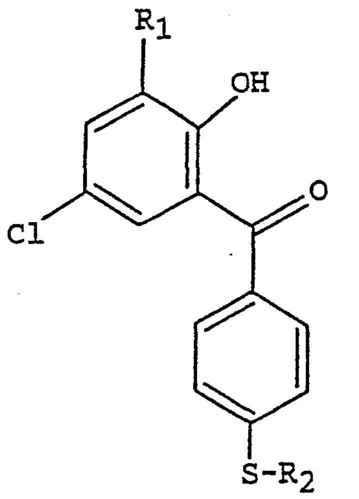

(III)

par exemple au moyen d'acide chloro-3 perbenzoïque, et finalement on condense le composé de formule III ou le composé oxydé de formule IV

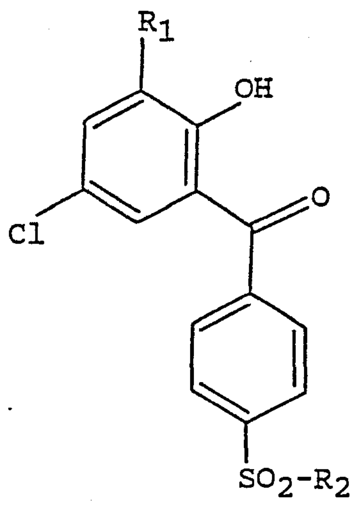

(IV)

avec la (1H-imidazolyl-2)méthanamine, à chaud et dans un solvant tel que l'éthanol.

7. Composé selon la revendication 1 pour la préparation de médicaments.

8. Composition pharmaceutique, caractérisée en ce qu'elle contient un composé selon la revendication 1, en association avec un excipient approprié.

Revendication pour les Etats Contractants Suivants : AT, ES, GR

Procédé de préparation d'un composé répondant à la formule générale I

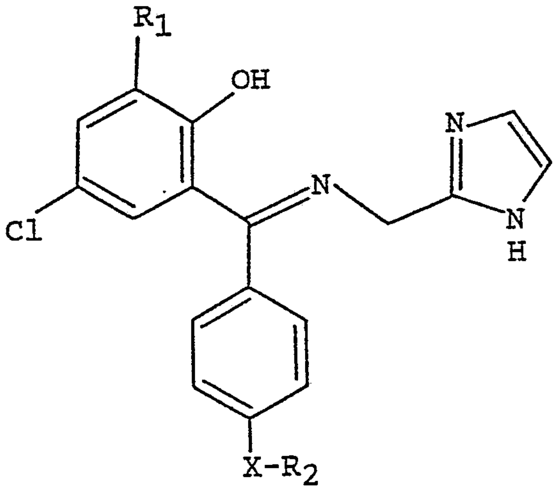

(I)

dans laquelle

$R_1$ représente un atome de chlore ou un groupe méthyle,

X représente un groupe thio ou sulfonyle, et

$R_2$ représente un groupe alkyle en $C_1$-$C_4$, allyle, cyclohexyle, pyridinyle-2, phényle éventuellement substitué par un groupe méthyle ou par un ou deux atomes d'halogène, ou encore benzyle éventuellement substitué par un atome d'halogène, par un groupe méthyle ou par un groupe méthoxy, procédé caractérisé en ce qu'on fait réagir une benzophénone de formule II

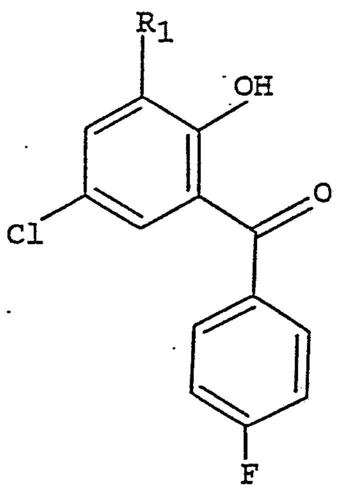

(II)

dans laquelle $R_1$ est tel que défini ci-dessus, avec le sel de sodium d'un mercaptan de formule $R_2$-SH, dans laquelle $R_2$ est tel que défini ci-dessus, à chaud et dans un solvant tel que le diméthylsulfoxyde, puis, si on désire un composé (I) où X représente un groupe sulfonyle, on oxyde l'intermédiaire de formule III

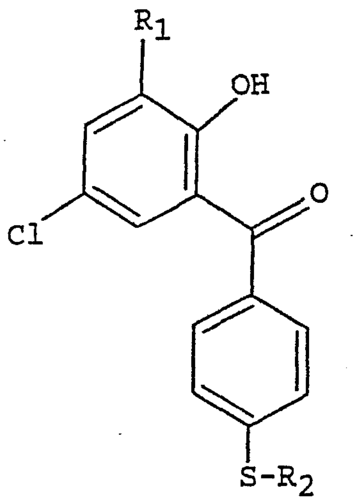

(III)

par exemple au moyen d'acide chloro-3 perbenzoïque, et finalement on condense le composé de formule III ou le composé oxydé de formule IV

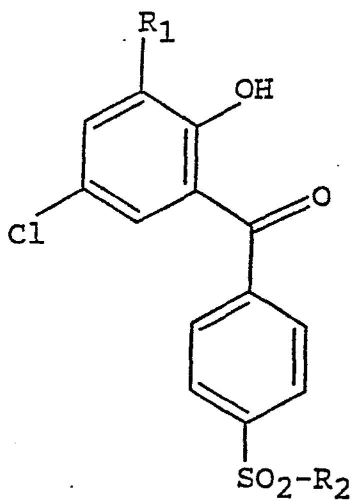

(IV)

avec la (1H-imidazolyl-2)méthanamine, à chaud et dans un solvant tel que l'éthanol.

Office européen des brevets

# RAPPORT DE RECHERCHE EUROPEENNE

Numero de la demande

EP 87 40 1352

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.4) |
|---|---|---|---|
| A | EP-A-0 133 061 (SYNTHELABO)<br>---- | | C 07 D 233/61<br>A 61 K 31/415 |
| A | FR-A-2 548 183 (SYNTHELABO)<br>----- | | |
| | | | DOMAINES TECHNIQUES RECHERCHES (Int. Cl.4) |
| | | | C 07 D 233/00<br>A 61 K 31/00 |

Le présent rapport de recherche a été établi pour toutes les revendications

| Lieu de la recherche | Date d'achèvement de la recherche | Examinateur |
|---|---|---|
| LA HAYE | 12-08-1987 | DE BUYSER I.A.F. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

OEB Form 1503 03 82